# EUROPEAN PATENT SPECIFICATION

(11) **EP 1 343 548 B1**
(45) Date of publication and mention of the grant of the patent: **28.03.2012**
(21) Application number: 01986998.1
(22) Date of filing: 16.11.2001
(51) Int. Cl.: A61L 31/16, A61L 31/12, A61L 29/16, A61L 29/12

(54) **METHOD AND APPARATUS FOR DELIVERY OF THERAPEUTIC FROM A DELIVERY MATRIX**
VORRICHTUNG UND VERFAHREN ZUR ABGABE VON THERAPEUTIKA
PROCEDE ET APPAREIL POUR DISTRIBUER UN PRODUIT THERAPEUTIQUE A PARTIR D'UNE MATRICE DE DISTRIBUTION

(30) Priority: 28.11.2000 US 723286
(43) Date of publication of application: 17.09.2003
(73) Proprietor: Boston Scientific Limited, St. Michael (BB)
(72) Inventor: NAIMARK, Wendy, Cambridge, MA 02138 (US); PALASIS, Maria, Wellesley, MA 02181 (US)
(74) Representative: Lang, Johannes
(86) International application number: PCT/US2001/043106
(87) International publication number: WO 2002/043788

(56) References cited:
- EP-A- 0 633 031
- WO-A-00/41647
- WO-A-99/12640
- US-A- 5 114 719

## Description

### Field Of The Invention

The present invention relates to a method for coating a medical device, a method for coating a medical device for insertion into the body of a patient and a medical device for insertion into the body of a patient.

### Background Of The Invention

The delivery of therapeutic materials may be done through various methods and means. Therapeutic may be injected into the body of a patient though the use of a needle and syringe, it may be absorbed through the skin of a patient via epidermal absorption patches, and it may also be released within the body, directly to a target site, through the use of implanted devices.

These implantable devices may be coated with therapeutic to be delivered. When a large molecular weigh therapeutic is sought to be delivered, emulsion based coating systems have been used in the past. In these emulsion based systems the device to be coated may be dipped into a solution containing microspheres of a carrier which encapsulates the therapeutic. These encapsulated microspheres retain the therapeutic and act to release it after the device is implanted in the body.

These emulsion based coating systems have specific limitations and inadequacies. For example, when the therapeutic to be delivered consists of genetically engineered material, the relatively delicate nature of the engineered material limits the choice of carriers that may be used to form the encapsulating microspheres. Similarly, in other situations, the solution itself may not be compatible with the medical device being coated. Consequently, in some emulsion based systems it may not be possible to optimize each of the various components of the entire drug delivery system. For example, while a specific therapeutic may be beneficial for treating a tumor the encapsulating carrier, which may be the most benign, may not be a plausible choice because it may not be compatible with the therapeutic. Similarly, the most effective therapeutic to treat a condition may not be plausible if it cannot be properly encapsulated. Thus, in certain circumstances a less than optimum carrier or therapeutic may be chosen, an undesirable result.

WO 00/41647 relates to sustained delivery of polyionic bioactive agents and describes coatings that comprise a matrix with a bioactive agent, where the bioactive agent is in a condensed from.

US-5,114,719 relates to extended drug delivery of small water-soluble molecules and uses a modified version of conventional solvent casting methods to apply coatings, which involves additional manufacturing steps, limitations on the solvents and freezing steps.

WO 99/12640 relates to coated particles, methods of making and using, where the matrix consists essentially of a nanostructured liquid phase and/or a nanostructured liquid crystalline phase, with an external coating of a nanolamellar crystalline material.

### Summary Of The Invention

The present invention provides an improved method for coating a medical device, an improved method of coating a medical device to be implanted in the body of a patient and an improved medical device for insertion into the body of a patient in accordance with the claims 1, 14, 25 and 26, resp. Embodiments of the invention are defined in the dependent claims.

In one embodiment a method of coating a medical device is provided. This embodiment includes applying a matrix to a medical device and applying a plurality of therapeutic particles to the medical device wherein the therapeutic particles may be restrained by the matrix and the matrix may be adapted to influence the release of the therapeutic particles from the matrix after the device is placed within the body of a patient

In another embodiment a medical device for insertion into the body of a patient is provided. This device includes a surface, a matrix covering the surface, and a plurality of therapeutic particles releasably combined with the matrix wherein the release rate of the therapeutic particles from the matrix may be regulated by the matrix.

In yet another embodiment a medical device for insertion into the body of a patient is provided. In this embodiment the medical device includes a surface and a means for releasably adhering a plurality of therapeutic particles to the surface of the medical device wherein the means covering the surface of the medical device may be adapted to regulate the release of the therapeutic particles into the body of the patient after the device has been inserted into the patient.

### Brief Description Of The Drawings

Fig. 1 is a front end transverse view of a stent coated in accordance with a first embodiment of the present invention.
Fig. 2 is an enlarged view along line 2-2 of the stent from Fig. 1.
Fig. 3 is a side view of the stent from Fig. 1.
Fig. 4 is a front view of two nozzles applying a coating to a medical device in accordance with a second embodiment of the present invention.
Fig. 5 is a top view of a medical device that has been coated in accordance with a third embodiment of the present invention.
Fig. 6 is a side view of four nozzles applying a coating to a stent in accordance with a fourth embodiment of the present invention.
Fig. 7 is an end view of a graft fabricated with a carrier polymer in accordance with a fifth embodiment of the present invention.

### Detailed Description

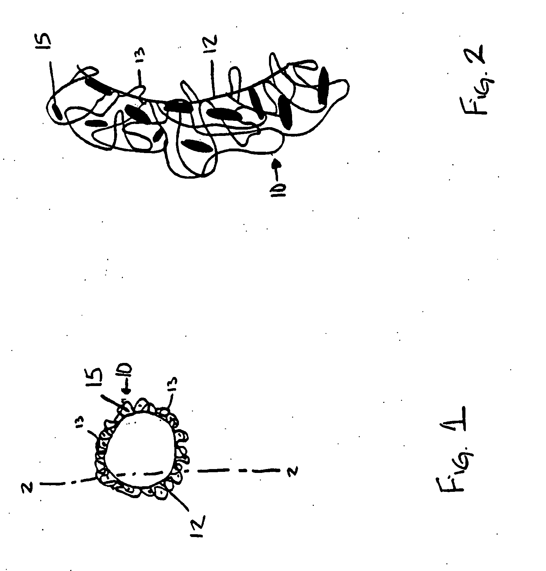

Fig. 1 is an end view of a tubular stent 12 that has been coated in accordance with one embodiment of the present invention. In this view the matrix 10 can be seen surrounding the entire circumference of the stent 12. As can also be seen therapeutic particles 15 are distributed throughout and restrained by the matrix. These therapeutic particles 15 may be nanoparticles of a large molecular weight therapeutic, they may preferably be helical in shape and may be less than 100 nm length. Alternatively, they may also be made of various other shapes and may range in size from 1 nm to 1000 nm or more. The matrix 10 in this embodiment, which may be a carrier or scaffolding polymer, may be designed to retain the therapeutic particles 15 within its woven fibers 13 while the stent 12 is outside of the body and then, after the stent 12 has been implanted into the body of a patient, to influence, regulate or govern the release of the therapeutic particles 15 from the matrix 10 to a target site within the body. While the matrix 10 may be principally responsible for governing the release of the therapeutic particles from the matrix other factors outside of the matrix such as the properties of the therapeutic and the location of the stent within the body may also influence the release of the therapeutic particles 15 from the stent 12. In other words, in use, the matrix 10, covering the stent 12, retains the therapeutic particles 15 within its fibers and generally governs the release of the therapeutic particles into the body even though other external factors may create secondary influences that affect the release of the therapeutic particles.

The release rate of the therapeutic particles 15 from the matrix 10 may be adjusted by modifying the various properties of the matrix 10 as well as by adjusting the properties of the therapeutic particles 15 relative to the matrix. These properties include the porosity of the matrix 10, which may be effected by adjusting the pressure placed on material being forced through a nozzle during the manufacturing process to create the matrix. The properties also include the diameters of the individual fibers 13 comprising the matrix, the size and shape of the therapeutic particles, and the quantity of therapeutic particles implanted into the carrier polymer. The diameter of the fibers may be adjusted by varying the distance from which the matrix is applied to the medical device, while the quantity of the particles implanted into the polymer may be effected by varying the same parameters mentioned above for modifying the matrix. In addition, the release rate from the matrix 10 may also be adjusted by applying several layers of the matrix 10 and therapeutic particles 15 on top of one another during the coating process.

In this embodiment as well as in other embodiments numerous macromolecular compounds may be used as the basis for the matrix 10 and for the therapeutic particles 15. Each of these macromolecular compounds, the matrix compound, and the therapeutic compound, which is partitioned into the therapeutic particles, may be chosen for their specific properties. In other words, both agents may be optimized for their specific purpose. For example, the matrix 10 may be a polymer specifically chosen for its durability, adaptability, and strength whereas the therapeutic used to create the therapeutic particles 15 may be a combination of a compound such as DNA and a condensing agent that facilitates or enhances the delivery of the DNA to a target site in the body.

Plausible matrix 10 compounds in these various embodiments include bioresorbable, biostable, hydrophobic, dissolvable macromolecules and polymers, and biopolymers such as proteins. The bioresorbable compounds may be polymers such as polylactic glycolic acid (PLGA) which can be resorbed into the body after therapeutic particles 15 imbedded within it have been released to a target site within the body. The biostable and hydrophilic compounds may be polymers such as SIBS (Styrene Isobutylene Styrene), which includes styrene and isobutylene styrene, which may not readily degrade within the body and may be used for sustained delivery of a therapeutic to a target site in the body. The dissolvable compounds could include biologically fabricated polymers (or biopolymers) such as proteins or polysaccharides. These dissolvable materials may be used for short term delivery applications that do not require post implantation invasive procedures such as with certain stent implantations.

The therapeutic particles 15 in this embodiment, as well as in other embodiments, may be composed of genetically engineered polymers and other macromolecules and may be chosen for their therapeutic qualities. These genetically engineered polymers may include oligonucleotides, plasmid deoxyribonucleic acid, recombinant viral vectors, and proteins. They may also include, for example, pharmaceutically active compounds, proteins, cells, oligonucleotides, ribozymes, anti-sense oligonucleotides, DNA compacting agents, gene/vector systems (i.e., any vehicle that allows for the uptake and expression of nucleic acids), nucleic acids (including, for example, recombinant nucleic acids; naked DNA, cDNA, RNA; genomic DNA, cDNA or RNA in a non-infectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoic acid; angiogenic and anti-angiogenic agents and factors; agents blocking smooth muscle cell proliferation such as rapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic / antiproliferative / anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as triclosan, cephalosporins, aminoglycosides, and nitorfurantoin; anesthetic agents such as lidocaine, bupivacaine, and ropivacaine; nitric oxide (NO) donors such as lisidomine, molsidomine, L-arginine, NO-protein adducts, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, Warafin sodium, Dicumarol, aspirin, prostaglandin inhibitors, platelet inhibitors and antiplatelet factors; vascular cell growth promoters such as growth factors, growth factor receptor antagonists, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vasoactive mechanisms; survival genes which protect against cell death, such as anti-apoptotic Bcl-2 family factors and Akt kinase; and combinations thereof. Cells can be of human origin (autologous or allogenic) or from an animal source (xenogenic), genetically engineered if desired to deliver proteins of interest at the injection site. The delivery mediated is formulated as needed to maintain cell function and viability. Any modifications are routinely made by one skilled in the art.

Polynucleotide sequences useful in practice of the invention include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides of the invention can also code for therapeutic proteins or polypeptides. A polypeptide is understood to be any translation product of a polynucleotide regardless of size, and whether glycosylated or not. Therapeutic proteins and polypeptides include as a primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor, hif-1, epidermal growth factor, transforming growth factor α and β, platelet-derived endothelial growth factor, platelet-derived growth factor, tumor necrosis factor a, hepatocyte growth factor and insulin like growth factor; growth factors; cell cycle inhibitors including CDK inhibitors; anti-restenosis agents, including p15, p16, p18, p19, p21, p27, p53, p57, Rb, nFkB and E2F decoys, thymidine kinase ("TK") and combinations thereof and other agents useful for interfering with cell proliferation, including agents for treating malignancies; and combinations thereof. Still other useful factors, which can be provided as polypeptides or as DNA encoding these polypeptides, include monocyte chemoattractant protein ("MCP-1"), laminins, and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA's encoding them.

In some circumstances, in order to enhance the uptake and transfer of these therapeutic particles 15 across cell membranes and to improve their cell recognition and tissue recognition qualities the therapeutic particles 15 may be condensed with a variety of polymer condensing agents including: poly-L-lysine, polyethylenimine, cationic lipids, protamines, histones, cyclodextran-containing polymers, and polyvinyl pyrrolidone. By using these various condensing agents the cell and tissue recognition capabilities of the therapeutic particles 15 may be modified to suit specifically tailored applications. In one illustrative example, when DNA is the macromolecule sought to be condensed and partitioned into the therapeutic particle 15, polymers, such as cyclodextran, polyethylene amine, and amphiphilic block co-polymers such as pluronics (BASF ^{™}) and formulations thereof, including L61 and F127 (Supratek™ formulations), may be used to facilitate the recognition capabilities of the therapeutic particles.

One advantage of this dual independent compound configuration (i.e., the therapeutic particles and the matrix) is that typically non-compatible polymers such as DNA, which is hydrophilic, and styrene and nitrobutylene styrene, which are hydrophobic, may, nevertheless, be utilized together on the same medical device because the independent compounds are fabricated separately and then meshed with one another on the surface of the medical device during the coating of the device to form the delivery system. Likewise, proteins and hydrophobic polymers, which require dissolution in organic solvents, may also be applied through these techniques as well as any combination where the matrix polymer used is soluble in organic solvents, such as with PLGA's, PEG-PLGA's and PU's.

Other examples of hydrophilic or hydrophobic polymers include, but are not limited to: polycarboxylic acids; cellulosic polymers, including cellulose acetate and cellulose nitrate; gelatin; polyvinylpyrrolidone; cross-linked polyvinylpyrrolidone; hydrogels; polyanhydrides including maleic anhydride polymers; polyamides; polyvinyl alcohols; copolymers of vinyl monomers such as EVA; polyvinyl ethers; polyvinyl aromatics; polyethylene oxides; laminins; glycosaminoglycans; polysaccharides; ethylene vinylacetate; polyesters including polyethylene terephthalate; polyacrylamides; polyethers; polyether sulfone; polycarbonate; polyalkylenes including polypropylene; polyethylene and high molecular weight polyethylene; halogenated polyalkylenes including polytetrafluoroethylene; polyurethanes; polyorthoesters; proteins; polypeptides; silicones, siloxane polymers; polylactic acid; polyglycolic acid; polycaprolactone; polyhydroxybutyrate valerate and blends and copolymers thereof as well as other biodegradable, bioabsorbable and biostable polymers and copolymers.

Fig. 2 is an enlarged view of a section taken along line 2-2 of Fig. 1. As can be seen in Fig. 2 the individual fibers 13 of the matrix 10 may be randomly located on the exterior surface of the stent 12 and may even penetrate inside the stent 12. As can also be seen in Fig. 2 the therapeutic particles 15 may be randomly located within the mesh formed by the individual fibers 13 of the matrix 10. As noted above, the density and size of these fibers 13, may be used to control the release rate of the therapeutic particles to a target site within the body of a patient.

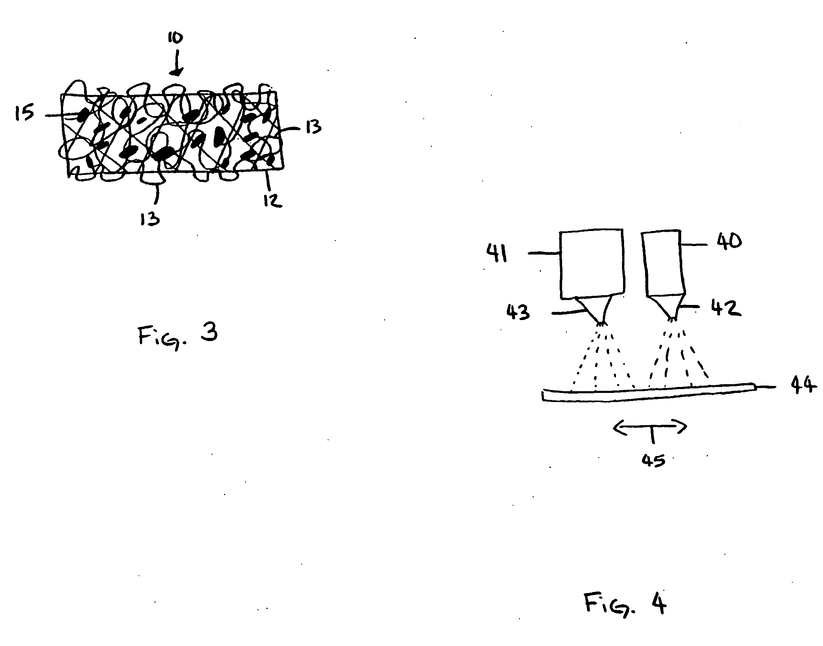

Fig. 3 is a side view of the stent from Fig. 1 in accordance with the first embodiment of the present invention. As can be seen in this view the entire outer surface of the stent 12 may be coated with both the therapeutic particles 15 and the matrix 10. Alternatively, rather than coating the entire stent 12, only portions of the stent 12 may be covered should a more localized delivery of the therapeutic particles 15 be required.

Fig. 4 is a side view of a medical device 44 being coated in accordance with a second embodiment of the present invention. As can be seen in Fig. 4 the medical device 44, which may be any one of numerous implantable devices, including a Meadox™ Mesh and a nitinol wire, may be coated with the matrix and the therapeutic particles during its manufacture. As is evident, by moving the device 44 back and forth, as indicated by arrow 45, the nozzles 42 and 43, which are being fed by hoppers 41 and 40, have access to and may spray a large portion of the surface of the device. In this embodiment, the first hopper, no. 40, may contain a matrix compound such as a polysaccharide, while the second hopper, no. 41, may contain a therapeutic compound such as genetically engineered DNA. Once loaded into the hoppers the two compounds may be placed under pressure so that they may be forced out through their respective hoppers and nozzles spraying the matrix material and the therapeutic at the surface of the medical device 44. The nozzles 42 and 43 in this embodiment may be adjusted to control the quantity and articulated size of the matrix and the therapeutic leaving the nozzles and reaching the medical device 44. Moreover, by adjusting the pressure in the hoppers, the concentration of each compound being ejected through the nozzle and deposited on the workpiece may be specifically tailored to achieve predetermined concentrations and configurations on the surface of the medical device. In addition, the feed rate of the medical device past the nozzles may also be adjusted to control the disposition rate and concentration of the matrix and the therapeutic particles onto the medical device 44.

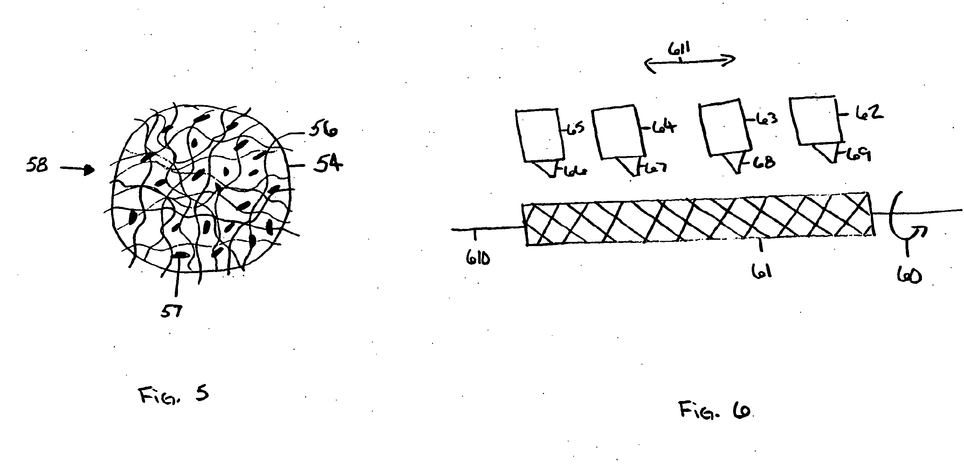

Fig. 5 is a top view of a therapeutic delivery patch made in accordance with a third alternative embodiment of the present invention. As can be seen this third embodiment contains therapeutic particles 57 implanted into a matrix 58, containing fibers 56. As is evident the fibers 56 of the matrix 58 in this embodiment may overhang the perimeter edges of the medical device 54. The medical device 54 in this embodiment may be coated on both sides with the matrix 58 and the therapeutic particles 57 or may simply be coated on a single side depending on the specific application of the medical device 54.

Fig. 6 is a side view of a stent 61 being coated in accordance with a fourth embodiment of the present invention. As can be seen the stent 61 in this embodiment may be mounted on a shaft 610 which may then be rotated as indicated by arrow 60. Located near the rotating stent 61 are several hoppers numbered 62-65 and several nozzles numbered 66-69 which may be connected to and in fluid communication with one another. As discussed above, the hoppers in this embodiment may be filed with the matrix compound or the therapeutic material and any condensing agent. Then, as discussed above, the various materials and compounds may be simultaneously forced from the nozzles and sprayed or otherwise deposited onto the stent 61 as it rotates. In this embodiment, rather than moving the stent 61 past the nozzles, the nozzles may be ganged together and may move back and forth along the stent 61 as indicated by arrow 611. In so doing the entire outer surface of a stent may be coated. The rate of movement, the process temperature, and the other process specifications may be governed by the individual materials being employed.

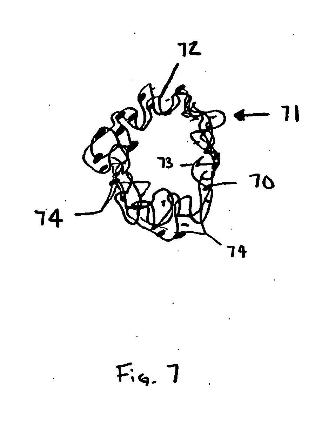

Fig. 7 is an end view of a graft made in accordance with a fifth embodiment of the present invention. As can be seen in Fig. 7 the graft 71 may be composed of a matrix 71 consisting of interwoven fibers 72 that have therapeutic particles 70 affixed to them. This embodiment differs from the previous embodiments as the matrix material has not been sprayed onto a tangible medical device but, rather, has been formed into a self-supporting shape - in this case a tubular graft. One advantage of this configuration is that the therapeutic particles 70 may be released from both the inside face 73 and outside face 74 of the matrix - as would be required in a graft installation. Another advantage of this configuration is that if the matrix is fabricated from bioabsorbable materials the graft may be implanted within the body for a temporary period until such time as it is absorbed into the body. Specifically, in use, due to its bioabsorbability, the graft may be surgically implanted, delivering a therapeutic for a specific time period, and then after it has served its purpose and has delivered the requisite amount of therapeutic, be reabsorbed into the body, thereby precluding the need for a second procedure to remove the graft.

In addition to the device applications described above the present invention may also be used to coat other devices for the delivery of therapeutic including: aneurysm filling coils, stents, therapeutic delivery balloons, coated trocars, implantable stomas, injection needles, coated therapeutic delivery fabrics, and coated therapeutic delivery meshes.

The various embodiments discussed above have numerous medicinal applications including: the correction of bladder incontinence; the reconstruction of vessel walls following an aneurysm; the treatment of any mammalian tissue or organ, whether treated in vivo or ex vivo including the heart, lungs, brain, liver, skeletal muscle, smooth muscle, kidney, bladder, intestines, stomach, pancreas, ovary, prostate, eye, tumors, cartilage and bone; the inducement or retardation of angiogenesis; the prevention or treatment of restenosis; the treatment of cardiomyopathy or other dysfunction of the heart; the treatment of Parkinsons, stroke or other dysfunction of the heart; the treatment of cystic fibrosis or other dysfunction of the lung; the treatment or retardation of malignant cell proliferation; the treatment of any malignancy; the regeneration of nerve, blood vessel or tissue in a particular tissue or organ; and, the long term delivery of therapeutics to any target area within the body.

## Claims

1. A method of coating a medical device comprising:
applying a matrix in an organic solvent to the medical device from a first delivery device; and,
applying a plurality of hydrophilic therapeutic particles to the medical device from a second delivery device,
wherein the therapeutic particles are restrained by the fibers of the matrix,
wherein the matrix influences the release of the therapeutic particles from the matrix when the medical device is placed within the body of a patient.

2. The method of claim 1 wherein the matrix is constructed from macromolecules.

3. The method of claim 1 wherein the matrix is a polymer.

4. The method of claim 1 wherein the matrix is a biostable polymer.

5. The method of claim 1 wherein the therapeutic particles are nanoparticles comprising a plurality of therapeutic macromolecules.

6. The method of claim 1 wherein the therapeutic particles contain a condensing agent.

7. The method of claim 6 wherein the condensing agent has cell recognition capabilities.

8. The method of claim 6 wherein the condensing agent has tissue recognition capabilities.

9. The method of claim 6 wherein the condensing agent increases the uptake of the therapeutic particles in the body of the patient.

10. The method of claim 1 wherein the therapeutic particles contain a genetically engineered polymer.

11. The method of claim 10 wherein the genetically engineered polymer contains a condensed nucleic acid.

12. The method of claim 1 wherein the release of the therapeutic particles from the matrix is governed by the porosity of the matrix.

13. The method of claim 1 wherein the matrix contains individual fibers and wherein the release of the therapeutic particles from the matrix is influenced by the individual fibers of the matrix.

14. A method of coating a medical device of claim 1, wherein the medical device is to be implanted in the body of a patient, wherein the matrix comprises a carrier polymer, comprising:
spraying a carrier polymer in an organic solvent at the exterior of the medical device from a first delivery device, wherein the carrier polymer comprises fibers; and
spraying hydrophilic therapeutic particles at the exterior of the medical device from a second delivery device, wherein the therapeutic particles are restrained by the fibers of the carrier polymer, the carrier polymer adapted to regulate the release of the therapeutic particles from the carrier polymer within the body of the patient.

15. The method of claim 14 wherein the carrier polymer and the therapeutic particles are concurrently sprayed at the medical device.

16. The method of claim 14 wherein the porosity of the carrier polymer regulates the release of the therapeutic particles from the carrier polymer.

17. The method of claim 14 wherein the fiber diameter of the carrier polymer regulates the release of the therapeutic particles from the carrier polymer.

18. The method of claim 14 wherein the quantity of therapeutic particles sprayed at the exterior of the medical device regulates the release rate of the therapeutic particles from the carrier polymer.

19. The method of claim 14 further comprising:
re-spraying the carrier polymer at the exterior of the medical device; and
re-spraying the therapeutic particles at the exterior of the medical device.

20. The method of claim 14 wherein the carrier polymer is a bioresorbable polymer.

21. The method of claim 14 wherein the therapeutic particles are combined with a condensing agent.

22. The method of claim 21 wherein the condensing agent has cell recognition capabilities.

23. The method of claim 21 wherein the condensing agent has tissue recognition capabilities.

24. The method of claim 21 wherein the therapeutic particles are nanoparticles of a macromolecule therapeutic.

25. A medical device for insertion into the body of a patient comprising:
a surface;
a matrix covering the surface; and,
a plurality of therapeutic particles releasably combined with the matrix,
the release rate of the therapeutic particles from the matrix regulated by the matrix, wherein the medical device is coated according to the method of claim 1.

26. The medical device of claim 25 wherein the therapeutic particles contain a condensing agent.

27. The medical device of claim 25 wherein the therapeutic particles are configured to have tissue recognition capabilities.

28. The medical device of claim 25 wherein the matrix is a bioresorbable polymer.

29. The medical device of claim 25 wherein the matrix is a biostable polymer.

30. The medical device of claim 25 wherein the matrix is dissolvable in the body of the patient.

31. The medical device of claim 25 wherein the therapeutic particles are a genetically engineered material.

32. A medical device for insertion into the body of a patient comprising:
a surface; and,
a matrix for releasably adhering a plurality of therapeutic particles to the surface of the medical device,
the matrix covering the surface of the medical device,
the matrix adapted to regulate the release of the therapeutic particles into the body of the patient at a predetermined rate,
wherein the medical devices is coated according to the method of claim 1.

## Patentansprüche

1. Verfahren zur Beschichtung einer medizinischen Vorrichtung umfassend:
Auftragen einer Matrix in einem organischen Lösungsmittel auf die medizinische Vorrichtung aus einer ersten Zuführungsvorrichtung; und
Auftragen einer Vielzahl hydrophiler therapeutischer Partikel auf die medizinische Vorrichtung aus einer zweiten Zuführungsvorrichtung, wobei die therapeutischen Partikel von den Fasern der Matrix zurückgehalten werden,
wobei die Matrix die Freisetzung der therapeutischen Partikel aus der Matrix beeinflusst, wenn die medizinische Vorrichtung in den Körper eines Patienten eingesetzt ist.

2. Verfahren nach Anspruch 1, wobei die Matrix aus Makromolekülen aufgebaut ist.

3. Verfahren nach Anspruch 1, wobei die Matrix ein Polymer ist.

4. Verfahren nach Anspruch 1, wobei die Matrix ein biostabiles Polymer ist.

5. Verfahren nach Anspruch 1, wobei die therapeutischen Partikel Nanopartikel sind, die eine Vielzahl therapeutischer Makromoleküle umfassen.

6. Verfahren nach Anspruch 1, wobei die therapeutischen Partikel ein Verdichtungsmittel enthalten.

7. Verfahren nach Anspruch 6, wobei das Verdichtungsmittel Zellerkennungs-Fähigkeiten aufweist.

8. Verfahren nach Anspruch 6, wobei das Verdichtungsmittel Gewebeerkennungs-Fähigkeiten aufweist.

9. Verfahren nach Anspruch 6, wobei das Verdichtungsmittel die Aufnahme therapeutischer Partikel in den Körper des Patienten erhöht.

10. Verfahren nach Anspruch 1, wobei die therapeutischen Partikel ein gentechnisch hergestelltes Polymer enthalten.

11. Verfahren nach Anspruch 10, wobei das gentechnisch hergestellte Polymer eine verdichtete Nukleinsäure enthält.

12. Verfahren nach Anspruch 1, wobei die Freisetzung der therapeutischen Partikel aus der Matrix durch die Porosität der Matrix gesteuert wird.

13. Verfahren nach Anspruch 1, wobei die Matrix individuelle Fasern enthält und wobei die Freisetzung der therapeutischen Partikel aus der Matrix durch die individuellen Fasern der Matrix beeinflusst wird.

14. Verfahren zur Beschichtung einer medizinischen Vorrichtung nach Anspruch 1, wobei die medizinische Vorrichtung in den Körper eines Patienten zu implantieren ist, wobei die Matrix ein Trägerpolymer umfasst, umfassend:
Aufsprühen eines Trägerpolymers in einem organischen Lösungsmittel auf die Außenseite einer medizinischen Vorrichtung aus einer ersten Zuführungsvorrichtung; und
Aufsprühen hydrophiler therapeutischer Partikel auf die Außenseite einer medizinischen Vorrichtung aus einer zweiten Zuführungsvorrichtung, wobei die therapeutischen Partikel durch die Fasern des Trägerpolymers zurückgehalten werden, und wobei das Trägerpolymer angepasst ist, die Freisetzung der therapeutischen Partikel aus dem Trägerpolymer innerhalb des Körpers des Patienten zu regulieren.

15. Verfahren nach Anspruch 14, wobei das Trägerpolymer und die therapeutischen Partikel gleichzeitig auf die medizinische Vorrichtung gesprüht werden.

16. Verfahren nach Anspruch 14, wobei die Porosität des Trägerpolymers die Freisetzung der therapeutischen Partikel aus dem Trägerpolymer reguliert.

17. Verfahren nach Anspruch 14, wobei der Faserdurchmesser des Trägerpolymers die Freisetzung der therapeutischen Partikel aus dem Trägerpolymer reguliert.

18. Verfahren nach Anspruch 14, wobei die Menge der auf die Außenseite der medizinischen Vorrichtung aufgesprühten therapeutischen Partikel die Freisetzungsrate der therapeutischen Partikel aus dem Trägerpolymer reguliert.

19. Verfahren nach Anspruch 14 des Weiteren umfassend:
das erneute Aufsprühen des Trägerpolymers auf die Außenseite der medizinischen Vorrichtung; und
das erneute Aufsprühen der therapeutischen Partikel auf die Außenseite der medizinischen Vorrichtung.

20. Verfahren nach Anspruch 14, wobei das Trägerpolymer ein bioresorbierbares Polymer ist.

21. Verfahren nach Anspruch 14, wobei die therapeutischen Partikel mit einem Verdichtungsmittel kombiniert werden.

22. Verfahren nach Anspruch 21, wobei das Verdichtungsmittel Zellerkennungs-Fähigkeiten aufweist.

23. Verfahren nach Anspruch 21, wobei das Verdichtungsmittel Gewebeerkennungs-Fähigkeiten aufweist.

24. Verfahren nach Anspruch 21, wobei die therapeutischen Partikel Nanopartikel eines makromolekularen Therapeutikums sind.

25. Medizinische Vorrichtung zur Insertion in den Körper eines Patienten umfassend:
eine Oberfläche;
eine die Oberfläche bedeckende Matrix; und
eine Vielzahl freisetzbarer, mit der Matrix kombinierter therapeutischer Partikel, wobei die Freisetzungsrate der therapeutischen Partikel aus der Matrix durch die Matrix reguliert wird, und wobei die medizinische Vorrichtung gemäß dem Verfahren nach Anspruch 1 beschichtet ist.

26. Medizinische Vorrichtung nach Anspruch 25, wobei die therapeutischen Partikel ein Verdichtungsmittel enthalten.

27. Medizinische Vorrichtung nach Anspruch 25, wobei die therapeutischen Partikel derart konfiguriert sind, dass sie Gewebeerkennungs-Fähigkeiten aufweisen.

28. Medizinische Vorrichtung nach Anspruch 25, wobei die Matrix ein bioresorbierbares Polymer ist.

29. Medizinische Vorrichtung nach Anspruch 25, wobei die Matrix ein biostabiles Polymer ist.

30. Medizinische Vorrichtung nach Anspruch 25, wobei die Matrix im Körper des Patienten auflösbar ist.

31. Medizinische Vorrichtung nach Anspruch 25, wobei die therapeutischen Partikel ein gentechnisch hergestelltes Material sind.

32. Medizinische Vorrichtung zur Insertion in den Körper eines Patienten umfassend:
eine Oberfläche; und,
eine Matrix zur freisetzbaren Anbringung einer Vielzahl therapeutischer Partikel auf der Oberfläche der medizinischen Vorrichtung, wobei die Matrix die Oberfläche der medizinischen Vorrichtung bedeckt, wobei die Matrix angepasst ist, die Freisetzung der therapeutischen Partikel in den Körper des Patienten mit einer vorgegebenen Rate zu regulieren,
wobei die medizinische Vorrichtung gemäß dem Verfahren nach Anspruch 1 beschichtet ist.

## Revendications

1. Un procédé de revêtement d'un dispositif médical, comprenant :
l'application au dispositif médical d'une matrice dans un solvant organique à partir d'un premier dispositif de délivrance ; et
l'application au dispositif médical d'une pluralité de particules thérapeutiques hydrophiles à partir d'un second dispositif de délivrance,
dans lequel les particules thérapeutiques sont retenues par les fibres de la matrice,
dans lequel la matrice influence la libération des particules thérapeutiques hors de la matrice lorsque le dispositif médical est placé dans le corps d'un patient.

2. Le procédé de la revendication 1, dans lequel la matrice est construite à partir de macromolécules.

3. Le procédé de la revendication 1, dans lequel la matrice est un polymère.

4. Le procédé de la revendication 1, dans lequel la matrice est un polymère biostable.

5. Le procédé de la revendication 1, dans lequel les particules thérapeutiques sont des nanoparticules comprenant une pluralité de macromolécules thérapeutiques.

6. Le procédé de la revendication 1, dans lequel les particules thérapeutiques contiennent un agent de condensation.

7. Le procédé de la revendication 6, dans lequel l'agent de condensation possède des propriétés de reconnaissance de cellules.

8. Le procédé de la revendication 6, dans lequel l'agent de condensation présente des propriétés de reconnaissance de tissus.

9. Le procédé de la revendication 6, dans lequel l'agent de condensation augmente l'absorption des particules thérapeutiques dans le corps du patient.

10. Le procédé de la revendication 1, dans lequel les particules thérapeutiques contiennent un polymère obtenu par génie génétique.

11. Le procédé de la revendication 10, dans lequel le polymère obtenu par génie génétique contient un acide nucléique condensé.

12. Le procédé de la revendication 1, dans lequel la libération des particules thérapeutiques hors de la matrice est gouvernée par la porosité de la matrice.

13. Le procédé de la revendication 1, dans lequel la matrice contient des fibres individuelles et dans lequel la libération des particules thérapeutiques hors de la matrice est influencée par les fibres individuelles de la matrice.

14. Un procédé de revêtement d'un dispositif médical selon la revendication 1, dans lequel le dispositif médical est destiné à être implanté dans le corps d'un patient, la matrice comprenant un polymère support, comprenant :
la pulvérisation à l'extérieur du dispositif médical d'un polymère support dans un solvant organique à partir d'un premier dispositif de délivrance, le polymère support comprenant des fibres ; et
la pulvérisation à l'extérieur du dispositif médical de particules thérapeutiques hydrophiles à partir d'un second dispositif de délivrance, les particules thérapeutiques étant retenues par les fibres du polymère support,
le polymère support étant apte à réguler la libération des particules thérapeutiques depuis le polymère support au sein du corps du patient.

15. Le procédé de la revendication 14, dans lequel le polymère support et les particules thérapeutiques sont pulvérisés concurremment sur le dispositif médical.

16. Le procédé de la revendication 14, dans lequel la porosité du polymère support régule la libération des particules thérapeutiques depuis le polymère support.

17. Le procédé de la revendication 14, dans lequel le diamètre des fibres du polymère support régule la libération des particules thérapeutiques depuis le polymère support.

18. Le procédé de la revendication 14, dans lequel la quantité de particules thérapeutiques pulvérisées sur l'extérieur du dispositif médical régule la vitesse de libération des particules thérapeutiques depuis le polymère support.

19. Le procédé de la revendication 14, comprenant en outre :
la re-pulvérisation du polymère support à l'extérieur du dispositif médical ; et
la re-pulvérisation des particules thérapeutiques à l'extérieur du dispositif médical.

20. Le procédé de la revendication 14, dans lequel le polymère support est un polymère biorésorbable.

21. Le procédé de la revendication 14, dans lequel les particules thérapeutiques sont combinées avec un agent de condensation.

22. Le procédé de la revendication 21, dans lequel l'agent de condensation présente des propriétés de reconnaissance de cellules.

23. Le procédé de la revendication 21, dans lequel l'agent de condensation possède des propriétés de reconnaissance des tissus.

24. Le procédé de la revendication 21, dans lequel les particules thérapeutiques sont des nanoparticules d'un agent thérapeutique macromoléculaire.

25. Un dispositif médical destiné à être inséré dans le corps d'un patient, comprenant :
une surface ;
une matrice recouvrant la surface ; et
une pluralité de particules thérapeutiques combinées de manière libérable avec la matrice,
la vitesse de libération des particules thérapeutiques depuis la matrice étant régulée par la matrice, le dispositif médical étant revêtu selon le procédé de la revendication 1.

26. Le dispositif médical de la revendication 25, dans lequel les particules thérapeutiques contiennent un agent de condensation.

27. Le dispositif médical de la revendication 25, dans lequel les particules thérapeutiques sont configurées pour présenter des propriétés de reconnaissance des tissus.

28. Le dispositif médical de la revendication 25, dans lequel la matrice est un polymère biorésorbable.

29. Le dispositif médical de la revendication 25, dans lequel la matrice est un polymère biostable.

30. Le dispositif médical de la revendication 25, dans lequel la matrice est soluble dans le corps du patient.

31. Le dispositif médical de la revendication 25, dans lequel les particules thérapeutiques sont un matériau obtenu par génie génétique.

32. Un dispositif médical destiné à être inséré dans le corps d'un patient, comprenant :
une surface ; et
une matrice pour retenir par adhérence de manière libérable une pluralité de particules thérapeutiques à la surface du dispositif médical,
la matrice couvrant la surface du dispositif médical,
la matrice étant apte à réguler la libération des particules thérapeutiques dans le corps du patient à une vitesse prédéterminée,
dans lequel le dispositif médical est revêtu selon le procédé de la revendication 1.
